Europäisches Patentamt

European Patent Office

Office européen des brevets

(19)

(11) EP 0 557 162 B1

(12) FASCICULE DE BREVET EUROPEEN

(45) Date de publication et mention
de la délivrance du brevet:
19.03.1997 Bulletin 1997/12

(51) Int Cl.⁶: **G02B 5/32**, G02B 5/20

(21) Numéro de dépôt: 93400329.4

(22) Date de dépôt: 09.02.1993

(54) **Filtre holographique de protection contre des rayonnements, notamment laser**

Holographisches Strahlenschutzfilter, besonders für Laser

Holographic radiation protection filter, particularly for lasers

(84) Etats contractants désignés:
DE FR GB SE

(30) Priorité: 18.02.1992 FR 9201812

(43) Date de publication de la demande:
25.08.1993 Bulletin 1993/34

(73) Titulaire: **SEXTANT AVIONIQUE**
**F-92360 Meudon-la-Forêt (FR)**

(72) Inventeurs:
• **Lepaih, Gérard**
**F-92402 Courbevoie Cedex (FR)**
• **Migozzi, Jean-Blaise**
**F-92402 Courbevoie Cedex (FR)**

(74) Mandataire: **Benoit, Monique et al**
**THOMSON-CSF-S.C.P.I.,**
**13, Avenue du Président**
**Salvador Allende**
**94117 Arcueil Cédex (FR)**

(56) Documents cités:
EP-A- 0 331 469          EP-A- 0 373 820
EP-A- 0 442 206          FR-A- 2 551 224
US-A- 4 601 533          US-A- 5 103 323

• PATENT ABSTRACTS OF JAPAN vol. 13, no. 112
(P-844) 17 mars 1989; & JP-A-63 287 986
• OPTICAL ENGINEERING, vol. 28, no. 6, juin 1989,
Bellingham US, pp. 609-615; J.M. TEDESCO:
'Holographic laser-protective filters and
eyewear'

## Description

La présente invention concerne un filtre holographique de protection contre des rayonnements, notamment laser. Elle s'applique particulièrement à la réalisation de lunettes ou de visières pour la protection efficace des yeux face à une illumination laser monoraie ou multiraies tout en offrant une bonne transmission sur le reste du spectre visible, de manière à garder un confort de vision au travers des lunettes ou des visières. Plus généralement, elle s'applique à la protection de tous capteurs physiques susceptibles d'être détruits ou endommagés par des rayonnements, émis par laser par exemple.

Il existe plusieurs solutions pour atténuer des rayonnements, par exemple par absorption d'une ou de plusieurs de leurs raies. Les absorbants classiques ne sont pas assez sélectifs et toute protection efficace altère considérablement la transmission du reste du spectre visible. Par exemple, pour se protéger d'une raie donnée et obtenir une atténuation correspondant à une densité supérieure à 5, l'ensemble du spectre visible devient très fortement atténué. La densité précitée exprimant l'atténuation de la raie signifie que, dans ce cas seulement une partie inférieure à $10^{-5}$ de l'énergie de la raie à atténuer est transmise. Plus généralement, une densité égale à n signifie que l'énergie transmise à travers l'absorbant est égale à $10^{-n}$ de l'énergie reçue par cet absorbant.

Les seuls absorbants sélectifs connus sont les absorbants "passe-rouge" ne filtrant que les raies de longueur d'onde inférieure à 520 nm environ, mais ces absorbants dégradent grandement la vision, le paysage ayant un aspect jaune, voire rouge.

Une autre solution consiste à utiliser des dispositifs de filtrage réalisés à base de couches minces ; celles-ci sont constituées par un empilements de couches métalliques ou diélectriques représentant une variation d'indice dont le but est de dévier et réfléchir la lumière. Dans de tels dispositifs, la largeur de la bande spectrale réfléchie est directement liée au domaine angulaire à protéger. Par exemple, s'il est envisagé de protéger un fort domaine angulaire, plus ou moins 45° par exemple, contre une raie laser (532 nm par exemple), il faut, pour un angle d'incidence donné, 30° par exemple, réfléchir une large bande spectrale, comprise entre 490 et 570 nm, ce qui détériore la vision. Si cette solution paraît suffisamment sélective au niveau de l'angle d'incidence, elle ne l'est pas au niveau de la longueur d'onde.

Des solutions connues permettent théoriquement de filtrer de façon sélective des rayons lumineux. Cependant, leurs réalisations pratiques ne donnent pas toujours les résultats escomptés et ne sont donc pas efficaces.

Un brevet US-4,601,533 présente un filtre constitué d'un support recouvert d'au moins deux hologrammes fixés entre eux par une colle 34.

Le but de l'invention est de pallier les inconvénients précités, notamment en permettant de filtrer de façon sélective et efficace un ou plusieurs rayons lumineux sur une grande plage angulaire tout en assurant une bonne transmission du reste du spectre visible.

A cet effet, l'invention a pour objet un filtre holographique tel que décrit par la revendication 1.

L'invention a pour principaux avantages qu'elle permet notamment une protection efficace de l'oeil ou de tout autre capteur face à des rayonnements laser et un confort de vision dans le cas d'application à des lunettes ou des visières par exemple.

D'autres caractéristiques et avantages de l'invention apparaîtront à l'aide de la description qui suit, faite en regard des dessins annexés qui représentent:

- la figure 1a, la position d'un oeil ou d'un capteur par rapport à un filtre ;
- la figure 1b, un exemple de réalisation d'un filtre ;
- la figure 2, un principe de méthode d'enregistrement d'hologramme pour la réalisation du filtre précité.
- la figure 3, un autre exemple de réalisation d'un filtre ;
- la figure 4, un principe de méthode d'enregistrement d'hologramme pour la réalisation du filtre précité ;
- la figure 5a, un exemple de réalisation d'un filtre selon l'invention comprenant une superposition d'hologrammes ;
- la figure 5b, un principe de méthode d'enregistrement d'hologramme pour la réalisation du filtre précité ;
- la figure 5c, des zones d'acceptance angulaire du filtre précité ;
- la figure 6, des réflexions entre des hologrammes d'un même filtre.

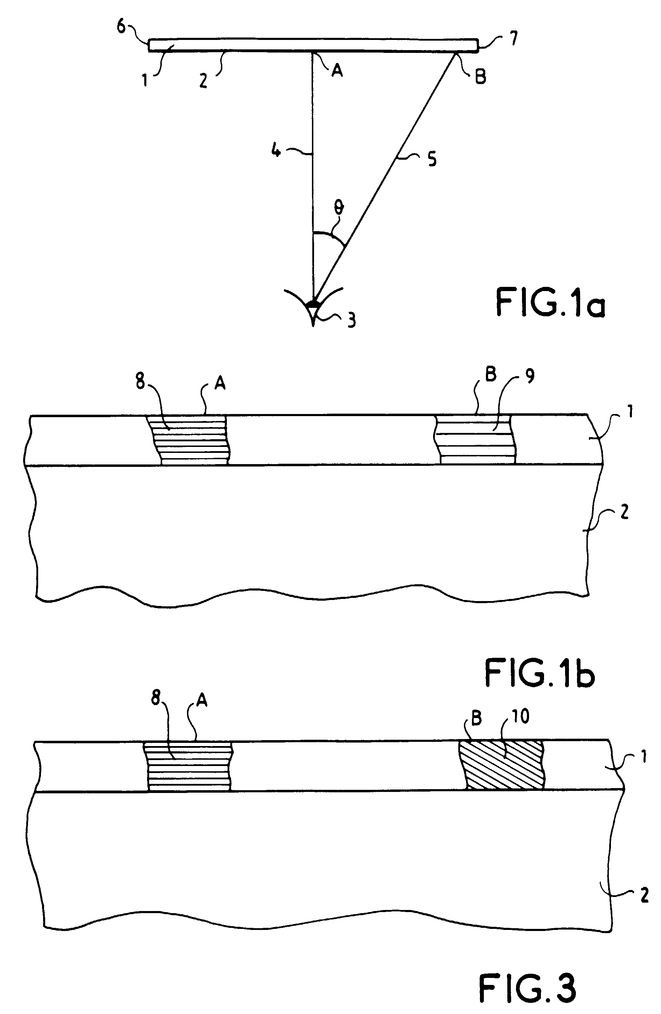

Les figures 1a, et 1b, illustrent un exemple de filtre dans le cas où l'oeil ou le capteur restent fixes par rapport au filtre. Sur la figure 1a, un support 1, transparent à la lumière, qui peut être plan, est recouvert par un hologramme 2. Cet hologramme 2 est vu par un oeil humain 3 ou un capteur par exemple, l'hologramme 2 est composé d'un empilement de strates. L'indice de réfraction de l'hologramme 2 présente une modulation d'indice. L'indice varie périodiquement suivant l'épaisseur du matériau d'enregistrement de l'hologramme, la période de variation constitue le pas des strates, cette variation peut par exemple être sinusoïdale.

Dans le but de filtrer une ou plusieurs raies optique de façon sélective, le support 1 recouvert de l'hologramme 2 étant à une distance sensiblement constante de l'oeil 3, la géométrie des strates varie suivant l'angle θ sous lequel elles sont vues par l'oeil 3.

La figure 1b présente une réalisation avec une variation de l'épaisseur du pas des strates de l'hologramme 2. Le support 1 et l'hologramme 2 sont principalement agrandis dans le sens de leurs épaisseurs par rapport à la figure 1a. La géométrie des strates est représentée telle qu'elle est par exemple à un point A et à un point B de l'hologramme. Le point A est vu par l'oeil 3 selon une direction 4 perpendiculaire au support 1 comme le montre la figure 1a alors que le point B est vu par l'oeil 3 selon une direction 5 faisant un angle θ avec la direction 4 précitée. Dans le cas de la figure 1b, la différence de géométrie entre les strates 8 au point A et les strates 9 au point B tient à la variation du pas entre celles-ci. Si λ désigne la longueur d'onde d'une raie à filtrer au point B et θ l'angle d'incidence de cette raie par rapport à la normale de l'hologramme 2 sur le support 1, le pas des strates de l'hologramme dans le matériau d'enregistrement holographique produisant la réflexion de la raie sur ce dernier est donné par la relation Bragg:

$$p = \frac{\lambda}{2\cos\theta} \qquad (1)$$

*p désignant le pas*

Ainsi le filtrage de la raie est obtenu par réflexion de celle-ci sur l'hologramme.
Par exemple, au point A, $p = \frac{\lambda}{2}$

Pour protéger l'oeil 3 ou tout autre capteur contre un rayonnement de longueur d'onde donnée dans toutes les directions suivant lesquelles l'oeil voit l'hologramme 2 entre les bords 6 et 7 de celui-ci, le pas des strates peut varier par exemple en fonction de l'angle θ sous lequel elles sont vues selon la relation (1) mentionnée précédemment.

Il serait possible de superposer plusieurs filtres de pas différents permettant de filtrer des longueurs d'ondes différentes sur des zones angulaires différentes.

Un avantage du filtre illustré par la figure 1b est notamment qu'il est aisé d'enregistrer l'hologramme 2 par des méthodes holographiques connues de l'homme de l'art. Il faut néanmoins, de préférence, que le pas des strates soit défini de façon suffisamment précise pour que le filtre réalisé selon l'invention soit suffisamment sélectif

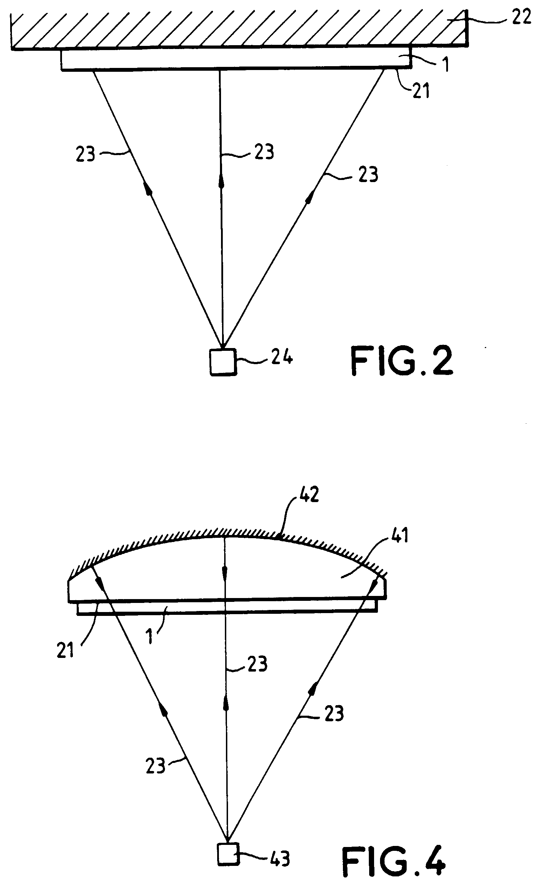

La figure 2 présente un mode de réalisation possible d'un tel hologramme à strates parallèles au support et à pas variable.

Pour améliorer la stabilité, le support 1 ayant une de ses faces recouverte d'un matériau photosensible 21 pour l'enregistrement de l'hologramme 2, une méthode possible consiste à le mettre au contact d'un miroir 22, la réflexion des rayons laser d'enregistrement 23 issus d'un point source 24 sur le miroir 22 générant des rayons interférant avec les précédents. Cette solution permet d'obtenir une seconde source d'émission parfaitement stable par rapport au support. L'angle d'incidence de chaque rayon d'enregistrement fixe le pas des strates et par voie de conséquence, le futur rayon incident filtré, à longueur d'onde donnée.

La figure 3 présente une autre configuration possible d'un filtre en illustrant une autre variation possible de géométrie des strates de l'hologramme 2 où l'orientation des strates varie. Dans ce mode de réalisation, le pas des strates au point A vu par l'oeil 3 suivant une direction normale par rapport au support 1 est par exemple identique au cas précédent, de la figure 1b, si la raie à filtrer est la même. Au point A, d'après la relation (1), le pas $p_A$ est donné par la relation suivante :

$$p_A = \frac{\lambda}{2} \qquad (2)$$

puisqu'en ce point cos θ = 1.

Au point B, les strates 1 ont le même pas que les strates 8 au point A, mais elles sont inclinées par rapport a ces dernières. Les strates au point B sont toutes parallèles entre elles mais elles sont inclinées de façon à être vues par l'oeil 3 suivant une direction perpendiculaire à leur plan d'inclinaison, raison pour laquelle le pas des strates au point B est toujours donné par la relation (2). Dans ce mode de réalisation possible, les strates de l'hologramme 2 sont orientées de façon à être vues par l'oeil 3 suivant une direction perpendiculaire à leur plan tangent, quelque soit l'endroit où elles se situent.

La configuration illustrée par la figure 1b offre l'avantage d'être simple à enregistrer et conduit notamment à un hologramme sans images parasites. Elle présente par contre une forte largeur spectrale en bord de champ, et permet un débattement de l'oeil ou du capteur limité.

Une méthode d'enregistrement des strates pour la réalisation de du mode de réalisation précité est illustrée par un schéma de principe présenté par la figure 4. Le support 1 est recouvert sur une de ses faces d'un matériau d'enregistrement photosensible 21, son autre face étant soumise à un rayonnement 23 de lumière cohérente. Le rayonnement 23 traverse le support 1 et le matériau d'enregistrement 21, celui-ci étant plaqué contre une lentille plan convexe 41 dont la face convexe 42 est traitée réfléchissante. La courbure est telle que tout rayon issu de la source 43 se réfléchit normalement sur la face convexe 42, correspondant à l'enregistrement d'une onde sphérique en autocollimation au centre d'un miroir concave. La sélection de la longueur d'onde filtrée peut se faire en jouant sur la longueur d'onde du rayonnement 23 qui doit être voisin de la longueur d'onde de protection.

Les types de filtre précédemment illustrés ne permettent pas de filtrer des longueurs d'ondes différentes sur des zones angulaires différentes et ne permettent donc notamment pas à un capteur ou à un oeil humain de rester protégé tout en étant mobile par rapport au filtre.

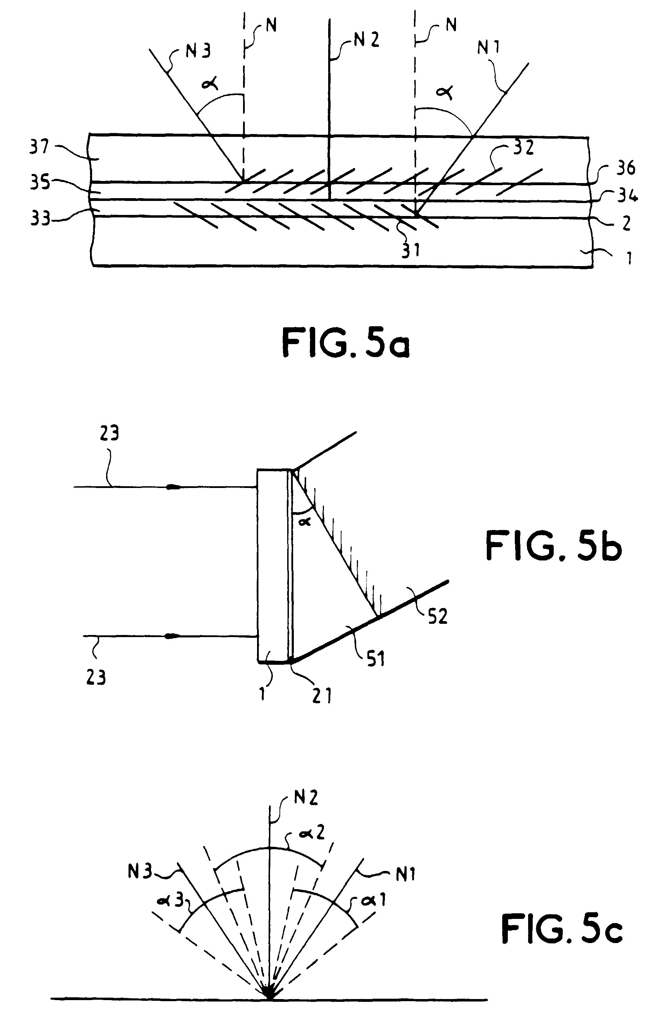

La figure 5a illustre une réalisation possible d'un filtre permettant à l'oeil 3 ou à tout autre capteur d'être protégé et mobile par rapport à l'hologramme, selon un axe coplanaire à la figure. Pour cela le filtre de la figure 5a comporte un premier support 1 recouvert d'un premier hologramme 2 dont les strates symbolisées par des traits 31 ont une inclinaison constante, la normale N1 à ces states faisant un angle α avec la normale N du support 1.

Une méthode d'enregistrement des strates inclinées à pas constant est illustrée par le schéma de principe présenté par la figure 5b. Le support 1 est recouvert sur une de ses faces d'un matériau d'enregistrement photosensible 21, son autre face étant soumise à un rayonnement 23 de lumière cohérente. Le rayonnement 23 traverse le support 1 et le matériau d'enregistrement 21, celui-ci étant plaqué contre un prisme 51 d'angle, au sommet, α. Le rayonnement se réfléchit selon un angle 2α contre un miroir 52, fixé sur le prisme 51. Les interférences créées par les rayons incidents et réfléchis à l'intérieur du prisme 51 sont enregistrées par le matériau 21 avec des strates inclinées d'un angle α par rapport au support.

Sur le premier hologramme 2 de la figure 5a est superposé un deuxième hologramme 34. Ces deux derniers sont par exemples fixés l'un à l'autre par une colle optique 33. Les strates du deuxième hologramme 34 sont parallèles au support 1, leur normale N2 est donc parallèle à la normale N du support 1 et leur pas est constant. Il s'obtient selon le principe de la figure 5b mais en supprimant le prisme, correspondant ainsi à l'enregistrement d'un faisceau collimaté sur un miroir. Le deuxième hologramme 34 est par exemple recouvert par un troisième hologramme 36. Ces deux derniers sont par exemple assemblés par une colle optique 35. L'hologramme 36, sur un support 37, a des strates inclinées symbolisées par les traits 32, identiques par exemples à celles du premier hologramme 2 à l'exception près que leur normale N3 fait un angle α avec la normale N du support 21 mais est symétrique à la normale N1 par rapport à cette normale N. Les longueurs d'onde filtrées selon la normale aux hologrammes 2, 34, 36 sont identiques.

Le mode de réalisation de la figure 5a permet de rendre mobile l'oeil 3 par rapport au filtre parallèlement au filtre dans le plan de la figure.

Chaque hologramme possède en pratique une zone angulaire autour de sa normale appelée zone d'acceptance angulaire où l'oeil ou tout autre capteur peut se mouvoir tout en restant protégé contre un rayonnement de longueur d'onde donnée. Ces zones font cependant un angle d'ouverture relativement faible et en conséquence, une seule zone offre peu de mobilité à l'oeil 3.

C'est le cas des figures 1b et 3 où un seul hologramme impose pratiquement à l'oeil de rester fixe par rapport au filtre pour être protégé. Le filtre de la figure 5a en augmentant le nombre de ces zones d'acceptance angulaire permet une mobilité de l'oeil 3 comme le montre la figure 5c.

La figure 5c illustre les zones d'acceptances angulaires α1, α2, α3 relatives respectivement aux hologrammes 2, 34, 36 filtrant par exemple chacun la même longueur d'onde. A condition que ces zones se chevauchent, comme c'est le cas sur la figure 5c l'oeil peut se déplacer dans la zone globale d'acceptance angulaire rassemblant ces trois zones angulaires α1, α2, α3 tout en restant protégé contre des rayonnements selon un axe coplanaire à la figure

Il serait possible d'augmenter le nombre d'hologrammes superposés pour augmenter la zone globale d'acceptance angulaire, ces hologramme ayant des strates plus ou moins inclinées.

Les exemples de réalisation de filtres présentés précédemment étaient monoraie, c'est à dire qu'ils filtraient le rayonnement dans une bande autour d'une longueur d'onde donnée, la bande pouvant être la plus étroite possible.

De manière analogue à l'exemple de réalisation de la figure 5b, il est possible de superposer plusieurs hologrammes, dans les limites acceptables de la qualité de vision. Mais dans ce cas, chaque hologramme filtre une longueur d'onde donnée.

Cela permet de réaliser un filtre multiraies, filtrant plusieurs longueurs d'ondes.

Les hologrammes peuvent alors être réalisés suivant une géométrie variable de leurs strates analogue au premier mode de réalisation de la figure 1b, c'est à dire par variation du pas de ces dernières, ou analogue au second mode de réalisation de la figure 3, c'est à dire par variation d'inclinaison des strates.

Il est possible de combiner ce dernier mode avec celui présenté par la figure 5b et réaliser un filtre multiraies.

Pour rendre efficace un filtre du type de celui illustré par la figure 5a et contenant au moins deux hologrammes superposés, la colle optique fixant les hologrammes entre eux est par exemple associée à un absorbant sélectif. Cet absorbant est alors sélectif pour les rayonnements à filtrer par exemple. Il peut être un colorant. Cet absorbant sélectif a un faible pouvoir d'atténuation et atténue donc faiblement l'énergie d'un rayonnement le traversant, cette énergie étant par exemple diminuée par un coefficient d'atténuation d'environ 1/1000 lors du passage du rayonnement dans l'absorbant. Cependant, bien qu'a priori sans effet, un tel absorbant peut contribuer à rendre le type de filtre précité efficace.

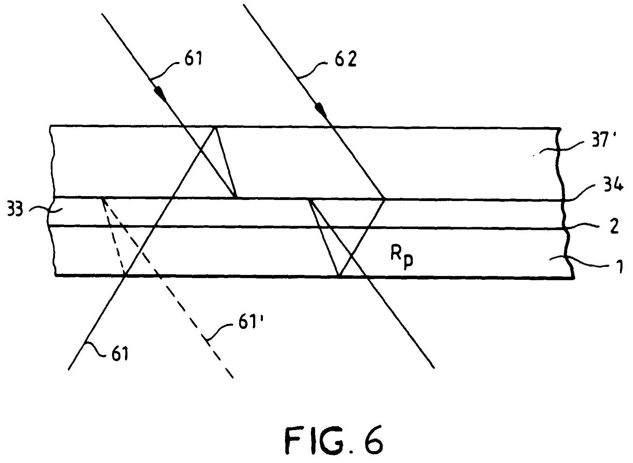

En effet, des rayonnements réfléchis par les hologrammes peuvent notamment se réfléchir de nouveau sur la face interne des supports comme l'illustre la figure 6, le rayonnement arrive ensuite sur les hologramme suivant un angle pour lequel il n'est par exemple pas filtré. Sur cette figure, un rayonnement 61 traverse un premier support 37', en verre par exemple, pour atteindre un premier hologramme 34 sur lequel il se réfléchit. Il est donc filtré par cet hologramme 34. Cependant le rayonnement réfléchi se réfléchit de nouveau sur la face interne du support 37'. Le rayonnement réfléchi rencontre alors le premier hologramme 34 sous un angle tel qu'il ne le filtre pas. De même, et pour la même raison il traverse un deuxième hologramme 2 fixé au premier par une colle optique 33. En sortie du deuxième hologramme 2, le rayonnement 61 peut se réfléchir sur la face interne d'un deuxième support 1, en verre par exemple, pour donner un rayonnement réfléchi 61', représenté en traits discontinus, qui se réfléchi par exemple sur le premier hologramme 34.

Un deuxième rayonnement 62 au lieu de se réfléchir peut donner un rayonnement diffracté $R_p$ au niveau du premier hologramme 34 susceptible de produire des images parasites. Ce rayonnement diffracté, peut par exemple traverser le deuxième hologramme 2, se réfléchir sur la face interne du deuxième support 1, puis se réfléchir sur le premier hologramme 34. D'autres exemples de réflexions multiples peuvent se produire entre les hologrammes, notamment successivement sur le premier 34 et le deuxième hologramme 2. A chaque passage d'un rayonnement dans la colle 33, si celle-ci est associée à un absorbant sélectif, notamment absorbant pour ce rayonnement, celui-ci est atténué par le coefficient d'atténuation de l'absorbant. Des réflexions multiples entraînent des passages multiples et l'effet de l'absorbant ne devient alors plus négligeable. Par ailleurs, plus le filtre contient de couches d'hologrammes, plus celui-ci devient performant et aussi plus efficace grâce notamment au couches de colles associées à un absorbant sélectif et intercalées entre les hologrammes, la combinaison du nombre de couches de colles et de réflexions multiples entre les hologrammes accroissant le pouvoir d'atténuation de l'absorbant sélectif associé à la colle. Cet absorbant sélectif permet notamment d'atténuer des rayonnements parasites, comme ceux illustrés par la figure 6 et obtenus à partir des rayonnements incidents 61, 62 sur le premier hologramme 34.

Les hologrammes peuvent être du type de ceux illustrés par les figures 1b ou 3, ou de tout autre type.

Les matériaux d'enregistrement des hologrammes peuvent être par exemple de la gélatine argentique, de la gélatine bichromatée ou un photopolymère, tous sensibles au rayonnement contre lequel il faut se protéger.

Les supports 1, 37, 37' peuvent être par exemple en verre, en plastique ou tous matériaux transparents aux ondes à transmettre et permettant la mise en oeuvre de l'enregistrement des hologrammes selon des méthodes connues de l'homme de l'art.

Enfin, un filtre selon l'invention peut être associé à une autre technique de filtre, notamment non holographique, constitué par exemple de couches minces.

## Revendications

1. Filtre holographique de protection d'un capteur (3) contre des rayonnements (61, 62), comprenant au moins un support (1) recouvert d'au moins deux hologrammes (2, 34), chaque hologramme étant constitué d'un empilement de strates (8, 9, 10) afin de filtrer le rayonnement dans une bande autour d'un longueur d'onde donnée, les hologrammes étant fixés entre eux par une colle optique (33), ledit filtre étant caractérisé en ce que la colle optique est associée à un absorbant sélectif pour lesdits rayonnements à filtrer, l'absorbant étant à faible pouvoir d'atténuation.

2. Filtre selon la revendication 1, caractérisé en ce que l'absorbant est un colorant.

3. Filtre selon l'une quelconque des revendications précédentes, caractérisé en ce que la géométrie des strates (8, 9, 10) d'un hologramme (2, 34) varie en fonction de l'angle ($\theta$) sous lequel elles sont vues par le capteur (3).

4. Filtre selon la revendication 3, caractérisé en ce que le pas des strates (8, 9) varie en fonction de l'angle ($\theta$) sous lequel elles sont vues par le capteur (3).

5. Filtre selon l'une quelconque des revendications 3 ou 4, caractérisé en ce que le pas des strates (8, 9) varie comme le rapport de la longueur d'onde à filtrer sur deux fois le cosinus de l'angle ($\theta$) sous lequel elles sont vues par le capteur (3).

6. Filtre selon la revendication 3, caractérisé en ce que l'inclinaison des strates (8, 10) par rapport à la normale du support (1) varie en fonction de l'angle ($\theta$) sous lequel elles sont vues par le capteur (3).

7. Filtre selon l'une quelconque des revendications 3 ou 6, caractérisé en ce que les strates (8, 10) sont orientées de façon à être vues par le capteur (3) suivant une direction perpendiculaire à leur plan tangent.

8. Filtre selon l'une quelconque des revendications précédentes, caractérisé en ce que les strates (8) vues par le capteur (3) sous un angle nul (A) sont perpendiculaires à la normale du support (1).

9. Filtre selon l'une quelconque des revendications précédentes, caractérisé en ce que la géométrie des strates est différente d'un hologramme à l'autre.

10. Filtre selon l'une quelconque des revendications précédentes, caractérisé en ce que le support (1) est en verre.

11. Filtre selon l'une quelconque des revendications 1 à 9, caractérisé en ce que le support (1) est en plastique.

## Patentansprüche

1. Holographisches Filter zum Schützen eines Sensors (3) gegen Strahlungen (61, 62), mit wenigstens einem Träger (1), der mit wenigstens zwei Hologrammen (2, 34) bedeckt ist, wobei jedes Hologramm durch einen Stapel von Schichten (8, 9, 10) gebildet ist, um die Strahlung in einem Band um eine gegebene Wellenlänge herum zu filtern, wobei die Hologramme aneinander durch einen optischen Klebstoff (33) befestigt sind, wobei das Filter dadurch gekennzeichnet ist, daß der optische Kleber einem selektiven Absorptionsmittel für die zu filternden Strahlungen zugeordnet ist, wobei das Absorptionsmittel ein geringes Dämpfungsvermögen hat.

2. Filter nach Anspruch 1, dadurch gekennzeichnet, daß das Absorptionsmittel ein Farbstoff ist.

3. Filter nach einem der vorhergehenden Ansprüche, dadurch gekennzeichnet, daß sich die geometrische Form der Schichten (8, 9, 10) eines Hologramms (2, 34) in Abhängigkeit von dem Winkel (θ) ändert, unter dem sie von dem Sensor (3) gesehen werden.

4. Filter nach Anspruch 3, dadurch gekennzeichnet, daß sich die Schrittweite der Schichten (8, 9) in Abhängigkeit von dem Winkel (θ) ändert, unter dem sie von dem Sensor (3) gesehen werden.

5. Filter nach einem der Ansprüche 3 und 4, dadurch gekennzeichnet, daß sich die Schrittweite der Schichten (8, 9) wie das Verhältnis der zu filternden Wellenlänge zu dem Zweifachen des Kosinus des Winkels (θ) ändert, unter dem sie von dem Sensor (3) gesehen werden.

6. Filter nach Anspruch 3, dadurch gekennzeichnet, daß sich die Neigung der Schichten (8, 10) bezüglich der Normalen auf den Träger (1) in Abhängigkeit von dem Winkel (θ) ändert, unter dem sie von dem Sensor (3) gesehen werden.

7. Filter nach einem der Ansprüche 3 und 6, dadurch gekennzeichnet, daß die Schichten (8, 10) so ausgerichtet sind, daß sie von dem Sensor (3) entlang einer zu ihrer Tangentialebene senkrechten Richtung gesehen werden.

8. Filter nach einem der vorhergehenden Ansprüche, dadurch gekennzeichnet, daß die von dem Sensor (3) unter einem Winkel Null (A) gesehenen Schichten (8) senkrecht zur Normalen auf den Träger (1) sind.

9. Filter nach einem der vorhergehenden Ansprüche, dadurch gekennzeichnet, daß sich die geometrische Form der Schichten von einem Hologramm zum anderen unterscheidet.

10. Filter nach einem der vorhergehenden Ansprüche, dadurch gekennzeichnet, daß der Träger (1) aus Glas besteht.

11. Filter nach einem der Ansprüche 1 bis 9, dadurch gekennzeichnet, daß der Träger (1) aus Kunststoff besteht.

## Claims

1. Holographic filter for protecting a sensor (3) from radiation (61, 62), comprising at least one support (1) covered with at least two holograms (2, 34), each hologram consisting of a stack of strata (8, 9, 10) in order to filter the radiation in a band around a given wavelength, the holograms being fixed to one another by an optical bonder (33), the said filter being characterized in that the optical bonder is combined with an absorber which is selective for the said radiation to be filtered, the absorber having a low attenuating power.

2. Filter according to Claim 1, characterized in that the absorber is a dye.

3. Filter according to either of the preceding claims, characterized in that the geometry of the strata (8, 9, 10) of a hologram (2, 34) varies as a function of the angle (θ) at which they are seen by the sensor (3).

4. Filter according to Claim 3, characterized in that the pitch of the strata (8, 9) varies as a function of the angle (3) at which they are seen by the sensor (3).

5. Filter according to either of Claims 3 and 4, characterized in that the pitch of the strata (8, 9) varies as the ratio of the wavelength to be filtered to twice the cosine of the angle (θ) at which they are seen by the sensor (3).

6. Filter according to Claim 3, characterized in that the inclination of the strata (8, 10) with respect to the normal to the support (1) varies as a function of the angle (θ) at which they are seen by the sensor (3).

7. Filter according to either of Claims 3 and 6, characterized in that the strata (8, 10) are oriented so as to be seen by the sensor (3) in a direction perpendicular to their tangent plane.

8. Filter according to any one of the preceding claims, characterized in that the strata (8) seen by the sensor (3) at a zero angle (A) are perpendicular to the normal to the support (1).

9. Filter according to any one of the preceding claims, characterized in that the geometry of the strata is different from one hologram to another.

10. Filter according to any one of the preceding claims, characterized in that the support (1) is made of glass.

11. Filter according to any one of Claims 1 to 9, characterized in that the support (1) is made of plastic.

FIG.1a

FIG.1b

FIG.3

8

FIG.2

FIG.4

FIG. 5a

FIG. 5b

FIG. 5c

FIG. 6